# EUROPÄISCHE PATENTSCHRIFT

(11) **EP 1 988 974 B1**
(45) Veröffentlichungstag und Bekanntmachung des Hinweises auf die Patenterteilung: **21.12.2011**
(21) Anmeldenummer: 07701903.2
(22) Anmeldetag: 28.02.2007
(51) Int. Cl.: A61Q 11/00, A61Q 11/02, A61K 8/22, A61K 8/02

(54) **ZAHNPASTA**
TOOTHPASTE
DENTIFRICE

(30) Priorität: 28.02.2006 CH 314062006
(43) Veröffentlichungstag der Anmeldung: 12.11.2008
(73) Patentinhaber: Swissdent Cosmetics AG, 8001 Zürich (CH)
(72) Erfinder: VELKOBORSKY, Vaclav, CH-8008 Zürich (CH)
(74) Vertreter: Grimm, Siegfried
(86) Internationale Anmeldenummer: PCT/CH2007/000103
(87) Internationale Veröffentlichungsnummer: WO 2007/098629

(56) Entgegenhaltungen:
- WO-A-2006/073822
- WO-A2-2006/069236
- GB-A- 2 054 375
- US-A- 4 897 258
- US-A- 5 403 578
- US-A- 5 423 337
- US-A- 5 496 542
- US-A- 5 693 334
- US-A1- 2002 064 541

## Beschreibung

Die vorliegende Erfindung betrifft eine Zahnpasta mit verbesserten Eigenschaften betreffend Reinigung und optischen Aufhellung von natürlichen Zähnen, künstlichen Zähnen und/oder Kunststofffüllungen von Zähnen; deren Herstellung und Verwendung.

GB2054375 beschreibt eine topische Zusammensetzung zur Behandlung von Akne und Seborrhoe, welche als aktive Komponente Benzoylperoxid und Natriumsulphosuccinat enthält.

Zahnpasten sind Zahnreinigungsmittel umfassend eine Zusammensetzungen aus (1) Scheuermittel, (2) Befeuchtungsmittel, (3) Bindemittel, (4) Wasser, (5) Geschmacksstoffen, (6) Detergens und (7) in kleinen Mengen Konservierungsmittel. Zusätzlich können sie Farbstoffe und weitere Additive enthalten, die einen positiven Effekt auf die Mund- und Zahnhygiene haben. Sie werden in erster Linie zur Schmutzentfernung mittels einer Zahnbürste eingesetzt. Zahnpasten dienen aber auch der Entfernung und Vorbeugung der Bildung von Plaques und Zahnstein, und der Regulation und Regeneration des Zahnfleisches. Deshalb enthalten Zahnpasten oft auch weitere Bestandteile wie Fluor, Vitamine, Kräuterextrakte usw.

Ein oft wünschenswerter kosmetischer Effekt von Zahnpasten ist auch das Aufhellen der Oberfläche von Zähnen, sowohl von natürlichen als auch von künstlichen Zähnen, sowie von Zahnfüllungen und Zahnprothesen. Hier stellt sich häufig das Problem, dass die Übergänge von natürlicher zu künstlicher Zahnsubstanz nicht völlig glatt sind, und dass kleinste Poren und Ritzen bereits zu Ablagerungen führen, die sich farblich von den Zähnen unterscheiden und schwer zu entfernen sind. Solche Stellen führen mit der Zeit zur Bildung von dunklen Rändern oder Flecken. Peroxide sind als chemische Aufheller bekannt und werden auch in Zahnpasten eingesetzt. Es handelt sich um reaktive Oxidationsmittel, die zwei hauptsächliche Nachteile haben: (1) sie sind toxisch, und (2) sie setzen - wenn sie mit Wasser in Verbindung kommen - Sauerstoff frei, was ein Problem bei der Abpackung und Haltbarkeit von Zahnpasten ist. Aus diesen Gründen ist in Zahnpasten im allgemeinen ein Gehalt von etwa 0.5 bis 5% Peroxid üblich (US5041280, US4603045, US4405599). US2002/0064541 beschreibt eine Formulierung enthaltend eine Peroxidverbindung in mikroverkapselter Form, wobei die Mikrokapseln vom Typ "Kern-Hülle" sind und eine spezifische Hülle aufweisen. Des weiteren wird die Verwendung solcher Mikrokapseln in Zahnbehandlungsmitteln offenbart. US5403578 beschreibt eine Zahnpasta enthaltend 3.5-11% Calciumperoxid in mikroverkapselter Form.

Es stellt sich daher die Aufgabe, eine Zahnpasta zur Verfügung zu stellen, die eine Zahn schonende, verbesserte Reinigung von natürlichen und künstlichen Zähnen, Zahnfüllungen und Zahnprothesen ermöglicht bei gleichzeitigem Aufhellen der Oberflächen einschliesslich der Ränder. Des weiteren stellt sich die Aufgabe, eine Zahnpasta zur Verfügung zu stellen, welche preiswert und aus einfachen Komponenten hergestellt werden kann.

Eine oder mehrere dieser Aufgaben werden von der Zahnpasta gemäss Patentanspruch 1 gelöst. Vorteilhafte Ausführungsformen sind in den abhängigen Ansprüchen und der Beschreibung angegeben. Weitere Aspekte der Erfindung sind in den unabhängigen Ansprüchen und in der Beschreibung angegeben.

Im Rahmen der vorliegenden Erfindung können allgemeine, bevorzugte und besonders bevorzugte Definitionen / Bereiche beliebig miteinander kombiniert werden. Ebenso können einzelne Definitionen entfallen.

Demgemäss betrifft die Erfindung in einem ersten Aspekt eine Zahnpasta, welche mikronisierte Bleichmittel wie Perborate und/oder Peroxide, vorzugsweise Peroxide, speziell bevorzugt Calciumperoxid enthält. Dadurch können die mikronisierten Bleichmittel ("Nanopartikel mit verbesserter Oberflächenaktivität") in kleinste Poren und Ritzen eindringen und dort länger wirksam bleiben, als dies mit nicht mikronisiertem Peroxid einer entsprechenden Konzentration der Fall ist. Dies führt zu einem verbesserten Bleicheffekt auf der gesamten, teilweise unregelmässigen Oberfläche der Zähne, insbesondere bei Übergängen von natürlichem zu künstlichem Zahnmaterial und bei den Rändern. Die Mikronisierung des Peroxids ermöglicht diesen Effekt selbst bei geringeren Konzentrationen. Neben dem Bleicheffekt hat das mikronisierte Peroxid auch generell einen positiven Effekt auf die Mundhygiene, da es toxisch ist für Bakterien, insbesondere für anaerobe Bakterien. Anaerobe Bakterien verursachen Karies und Mundgeruch. Sie befinden sich nicht nur in den Plaques, sondern auch in Belägen des Rachens und der Mundhöhle, insbesondere der Zunge. Ausserdem wird durch mikronisiertes Peroxid eine entzündungshemmende Wirkung erzielt, was insbesondere zu verminderter Zahnfleischblutung führt.

Weiterhin wurde gefunden, dass die erfindungsgemässe Zahnpaste, welche unverkapselte mikronisierte Peroxidverbindungen enthält, eine überraschende Stablilität in der Verpackung aufweist und gleichzeitig eine sehr gute Wirkung bei der Benutzung zeigt.

In einer vorteilhaften Ausführungsform betrifft die Erfindung eine Zahnpasta zur Reinigung und optischen Aufhellung von natürlichen und/oder künstlichen Zähnen und Zahnfüllungen, dadurch gekennzeichnet, dass sie mikronisiertes Peroxid enthält.

In einer weiteren vorteilhaften Ausführungsform betrifft die Erfindung Zahnpasta enthaltend eine oder mehrere mikronisierte unverkapselte Peroxid-Verbindungen.

Bevorzugt beträgt nach Mikronisierung der Durchmesser von 50% der Teilchen weniger als 2 bis 3 Mikrometer, wobei mindestens 90% der Teilchen einen Durchmesser von weniger als 5 bis 7 Mikrometer haben. Diese Teilchen besitzen insgesamt eine grössere Oberfläche und deshalb mehr aktive Stellen und erlauben eine bessere Bleichung der Zähne während der Anwendung. Ohne sich an eine Theorie zu binden, wird davon ausgegangen, dass die Zeit des Kontakts eines mikronisierten Peroxid Partikels mit dem Zahn vergrössert wird, indem dieses Nanopartikel in die Poren eindringen und dort verweilen kann, während nicht mikronisiertes Peroxid rasch wieder vom Zahn weg diffundiert. In einer bevorzugten Ausführungsform wird besagte mikronisierte Peroxidverbindung mittels einer Jet-Mill erzeugt.

Wie aus den vorstehend genannten Ausführungen deutlich wird, ist die eingesetzte Peroxidverbindung unverkapselt.

Im Rahmen der vorliegenden Erfindung werden die Begriffe "Peroxid" und "Peroxid-Verbindung" synonym benutzt. Generell können alle Peroxid-Verbindungen verwendet werden, die im Zusammenhang mit Zahnpaste und / oder Mundhygiene bekannt sind und sich als Feststoff mikronisieren lassen. Das für die Erfindung bevorzugte Peroxid ist Calciumperoxid. Es liegt erfindungsgemäss in einer Konzentration von über etwa 0.01% vor. Die untere Grenze der Peroxid Konzentration liegt vorzugsweise bei etwa 0.05%, speziell bevorzugt bei 0.1%. Normalerweise beträgt die Peroxid Konzentration nicht mehr als etwa 10%, bevorzugt nicht mehr als 5%, und speziell bevorzugt nicht mehr als 2% oder die für den jeweiligen Zweck zugelassene Konzentration. (Die Schweizer Norm für Peroxid in Kosmetika liegt bei 0.1%.) Unter % sind immer Gewichtsprozente bezogen auf O₂²⁻ zu verstehen.

Da viele Zahnablagerungen Proteine enthalten, stellt die Erfindung in einer weiteren Ausführungsform auch eine Zahnpasta mit mikronisiertem Peroxid zur Verfügung, die zusätzlich Enzyme mit proteolytischer Aktivität enthält. Solche Proteasen helfen, die proteinhaltigen Ablagerungen auf den Zähnen zu zerstören, womit ein verbesserter Zugang der Peroxid Partikel zur unter den Ablagerungen liegenden Zahnoberfläche ermöglicht wird. Ebenfalls werden bakterielle Ablagerungen, auch Plaques genannt, durch solche Proteasen angegriffen, weil die bakteriellen Oberflächenproteine Substrate dieser Proteasen sind. Zudem ist Peroxid selbst toxisch für viele Bakterien. Es ergibt sich somit ein synergistischer Effekt bei der Verwendung von mikronisiertem Peroxid, insbesondere Calciumperoxid, zusammen mit Proteasen, weil eine verbesserte Zahnbleichung durch eine gleichzeitige Verminderung der bakteriellen und proteinhaltigen Ablagerungen ermöglicht wird.

Als Proteasen werden in der vorliegenden Erfindung die Cysteinproteasen bevorzugt, insbesondere Papain und Bromelain. Papain ist eine Sulfhydrylprotease mit geringer Substratspezifität die aus dem Latexsaft von *Carica papaya* gewonnen wird. Bromelain ist eine Zusammensetzung, die aus der Ananas gewonnen wird und mehrere Cysteinproteasen enthält. Papain und Bromelain können in der Zahnpasta einzeln vorhanden sein. Zusammen und in Kombination mit mikronisiertem Peroxid haben diese Proteasen eine vorteilhafte Wirkung auf die Zahnbleichung, weshalb in einer bevorzugten Ausführung der vorliegenden Erfindung alle drei Komponenten in der Zahnpasta enthalten sind.

Die Zahnpasta der vorliegenden Erfindung umfasst Pasten oder Gele mit einer für die Zahnreinigung geeigneten Konsistenz, die weder zu fest noch zu flüssig sein darf. Eine solche Zahnpasta enthält neben Wasser mindestens ein Scheuermittel, bevorzugt Kieselsäure (SiO₂) stärker bevorzugt Sident 8 und Sident 22 S (Degussa AG) im Verhältnis 9:5.

In einer vorteilhaften Ausführungsform enthält die erfindungsgemässe Zahnpasta Befeuchtungsmittel wie zum Bespiel Glycerin, Sorbitol, Propylenglycol, Polyglycole, bevorzugt Glycerin und/oder Sorbitol.

In einer vorteilhaften Ausführungsform enthält die erfindungsgemässe Zahnpasta Bindemittel, zum Beispiel Gele pflanzlichen Urspruchs wie Cellulose, Celluloseether oder Pectin, Xanthan, Carbomere oder Kieselsäuren, oder Polysaccharide aus Algen, bekannt als Carrageenan. Für die vorliegende Erfindung bevorzugt verwendet wird Carrageenan.

In einer vorteilhaften Ausführungsform enthält die erfindungsgemässe Zahnpasta mindestens ein Detergens aus der Gruppe umfassend Betaine, Ethersulfate und Sarcosinate. Bevorzugt ist Cocamidopropyl-Betaine, vorzugsweise in einer Konzentration von ungefähr 2%.

Nicht notwendigerweise, aber bevorzugt, enthält die erfindungsgemässe Zahnpasta auch einen oder mehrere Inhaltsstoffe aus der Gruppe der Konservierungsmittel, z.B. Methylparaben; der Geschmacks- und Aromastoffe; der fluorhaltigen Verbindungen, vorzugsweise als Natrium Monofluorophosphat; der kosmetischen Farbstoffe; der Vitamine, vorzugsweise Vitamine E; der Coenzyme, vorzugsweise Ubichinon und ggf. weitere Zusatzstoffe.

In einer vorteilhaften Ausführungsform enthält die erfindungsgemässe Zahnpasta Vitamin E und Ubichinon.

In einer bevorzugten Ausführungsform betrifft die Erfindung daher eine Zahnpaste mit einem ph-Wert im Bereich von 5 bis 8, speziell bevorzugt zwischen 6.5 und 7.5. Es ist auch wünschenswert, dass die erfindungsgemässe Zahnpasta einen annähernd neutralen pH hat.

In einem **weiteren Aspekt** betrifft die vorliegende Erfindung die Herstellung einer Zahnpasta wie vorstehend beschrieben. Die Herstellung der erfindungsgemässen Zahnpasta erfolgt durch Zusammenfügen und Mischen der Bestandteile in einer bestimmten Reihenfolge. Zuerst werden Zusatzstoffe wie Enzyme, Co-enzyme, Vitamine, Süssstoff und Fluor einzeln zu einer Mischung aus Wasser, Konservierungsmittel und Befeuchtungsmittel gegeben und darin gelöst. Nicht wasserlösliche Bestandteile wie z.B. Ubichinon oder Vitamin E können dabei in Form von Liposomen beigefügt werden. Danach wird Bindemittel zugegeben, das erhaltene Gemisch wird quellen gelassen und homogenisiert. Anschliessend erfolgt die Zugabe der Scheuermittel und des mikronisierten Peroxides. Schliesslich werden Aroma- und Farbstoff eingearbeitet. Ganz am Schluss wird das Detergens zugegeben, und das erhaltene Gemisch homogenisiert. Die Erfindung betrifft daher ein Verfahren zur Herstellung von Zahnpasta umfassend die Schritte i) Herstellen einer Lösung aus Wasser, Befeuchtungsmittel und Konservierungmittel und löslicher Zusatzmittel der Gruppe Enzyme, Coenzyme, Vitamine, Süssstoffe, fluorhaltige Verbindungen wobei unlösliche Verbindungen in Form von Liposomen zugegeben werden können; ii)Zugabe von Bindemittel mit anschliessendem quellen und homogenisieren; iii) Zugabe von Scheuermittel und mikronisierter Peroxidverbindung und homogenisieren; iv) Zugabe von Aroma- und Farbstoffen und homogenisieren.

In einem **weiteren Aspekt** betrifft die vorliegende Erfindung die Verwendung einer Zahnpasta zur Reinigung und optischen Aufhellung von natürlichen und/oder künstlichen Zähnen und Zahnfüllungen.

Weitere bevorzugte Ausführungen der Erfindung ergeben sich aus den abhängigen Ansprüchen sowie aus den folgenden Ausführungsbeispielen.

### Bsp.1: Mikronisierung von Calciumperoxid

Die Mikronisierung erfolgt mit Calciumperoxid, das als Pulver mit einen Anteil von mindestens 60% Calciumperoxid erhältlich ist. Die Mikronisierung ist ein trockenes Verfahren, das kein Lösungsmittel erfordert. In einer MC JETMILL^{®} Luftstrahlmühle (Jetpharma SA) werden die einzelnen Calciumperoxid Kristalle durch komprimiertes Gas, entweder Stickstoff oder Luft, beschleunigt, sodass die Teilchen aufeinander prallen und in kleiner und immer kleiner werdende Partikel zerfallen. Die Partikelgrösse kann über bekannte analytische Verfahren bestimmt werden, vorzugsweise mit einem Lasergerät zur Partikelgrössenmessung (Malvern Mastersizer MicroPlus, Grössenbereich 0.05-556 Mikrometer). Die Korngrösse, die durch die Mikronisierung erreicht werden kann, beträgt bei 50% der Partikel weniger als 2-3 Mikrometer und für 90% der Partikel weniger als 5-7 Mikrometer.

### Bsp.2: Zahnpasta mit mikronisiertem Calciumperoxid

| | |
|---|---|
| 100 kg der Zahnpasta wird folgendermassen hergestellt: | |
| zu | |
| Wasser, keimfrei | 51.3657 kg |
| Werden der Reihe nach folgende Komponenten zugegeben | |
| Methylparaben Ph. Eur. | 0.0540 kg |
| Glycerin 99.5% | 10.0000 kg |
| Nano-Lipobelle H-EQ10 (Liposomen beladen mit 10% Vitamin E und 5% Ubichinon) | 0.9000 kg |
| Sorbitol 70% | 7.0000 kg |
| Zinkcitrat-hydrat | 0.8700 kg |
| Natriumsaccharin | 0.0660 kg |
| Papain | 0.2000 kg |
| Bromelain | 0.2000 kg |
| Phoskadent NA 211 (Natruim-Monofluorophosphat) | 0.8600 kg |
| Satiagel UTH 18 (Carrageenan) | 0.8700 kg |

Diese Zusammensetzung wird für mindestens 30 Minuten homogenisiert und quellen gelassen. Danach werden weiter zugegeben und eingearbeitet

| | |
|---|---|
| Prestige Sparkling Green 3518 enthaltend Mica, Titandioxid (CI77891) und Zinnoxid | 0.8000 kg |
| Martinal ON-310 (Aluminiumhydroxid) | 3.2600 kg |
| Sident 8 (Silica) | 12.6000 kg |
| Sident 22 S Kieselsäure | 7.0000 kg |
| Mikronisiertes Calciumperoxid | 0.4500 kg |
| Pfefferminzöl (natürlich) | 1.5000 kg |
| Pistaziengrün (CI2051 + CI19140) | 0.0043 kg |
| Zum Schluss wird | |
| Tego Betain L7 | 2.0000 kg |

langsam über einen Trichter eingesaugt, 2 Stunden stehen gelassen, danach homogeisiert und auf -1bar vakuumisiert.

### Bsp.3: Studie zur Bestimmung der Zahnaufhellung

### Allgemeines Vorgehen:

Bei zufällig ausgewählten Probanden wird zu Beginn sowie nach einiger zeit, geeigneterweise nach zwei Wochen der Studie die Zahnfarbe der oberen und unteren Schneidezähne mittels eines Farbmessgeräts (Spectroshade) ermittelt. In den letzten vier Wochen vor Studienbeginn soll keine professionelle Zahnreinigung durchgeführt und keine Zahncreme zur Beseitigung von Zahnverfärbungen benutzt worden sein.

### Studienablauf:

Bei der Eingangsuntersuchung wird bei jedem Probanden die Zahnfarbe der beiden oberen und der beiden unteren Schneidezähne zuerst mit Spectroshade bestimmt. Zusätzlich wird die Fläche mit der intensivsten Verfärbung ausgewählt. Diese Fläche wird in eine Zahnskizze des Probandenprotokolls eingezeichnet.

Die aus der Spectroshade Messung ermittelten Zahnfarben werden entsprechend ihrer Helligkeit in eine Vitapan®-Skala mit Werten von 1 bis 16 überführt. So erhält die hellste Zahnfarbe B1 den Wert 1 und die dunkelste Farbe C4 den Wert 16 (Tab. I).

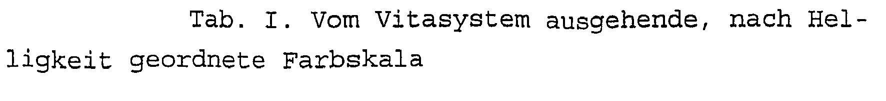

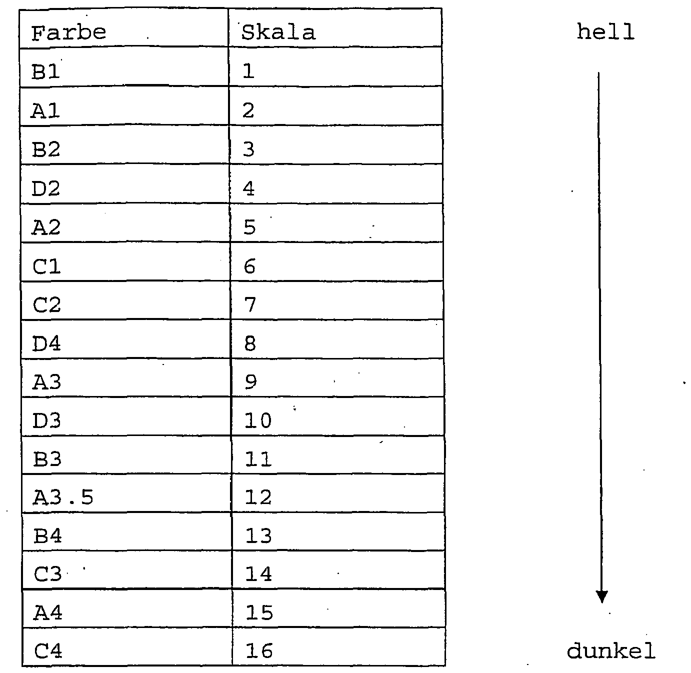

Die Summe der Werte der vier gemessenen Zähne wird durch vier geteilt, so dass sich pro Patient jeweils ein Durchschnittswert ergibt.

Die Probanden werden angewiesen, wie bisher gewohnt die Zähne mit der Testzahncreme zu putzen.

Nach geeigneter Zeit, üblicherweise zwei Wochen, werden die oben genannten Untersuchungen wiederholt. Zusätzlich können die Probanden mittels eines Fragebogens ebenfalls um eine subjektive Beurteilung der Zahnpasta hinsichtlich des Aufhellungseffektes gebeten werden. Sinnvollerweise werden sie auch bezüglich der Sicherheit und Verträglichkeit der Paste befragt.

### Resultate aus Voruntersuchungen

30 Probanden beendeten die klinische Studie. Nach 2 Wochen zeigte sich sowohl bei der visuellen als auch bei der SPECTROSHADE Bestimmung ein deutlicher Helligkeitsunterschied im Vergleich zum Studienbeginn.

So ergab sich am Ende der Studie, d.h. nach zwei Wochen eine Aufhellung der Zähne von mindestens 2, oft mehr als 4 Helligkeitsstufen.

Bei der Auswertung des Patientenfragebogens hatten 85% der Teilnehmer das Gefühl, die Zähne seien wesentlich bezw. etwas heller geworden. 15% sahen keinen Unterschied. Ebenso wurde eine Abnahme des Mundgeruchs festgestellt, sowie verminderte Zahnfleischblutung.

## Patentansprüche

1. Zahnpasta enthaltend Wasser; mindestens ein Scheuermittel und eine oder mehrere mikronisierte, unverkapselte Peroxid-Verbindungen, wobei 50% der Teilchen besagter Peroxid-Verbindungen einen Durchmesser von weniger als 3 Mikrometer und mindestens 90% der Teilchen besagter Peroxid-Verbindungen einen Durchmesser von weniger als 7 Mikrometer haben.

2. Zahnpasta gemäss Anspruch 1, **dadurch gekennzeichnet, dass** 50% besagter Teilchen einen Durchmesser von weniger als 2 Mikrometer, und mindestens 90% besagter Teilchen einen Durchmesser von weniger als 5 Mikrometer haben.

3. Zahnpasta gemäss Anspruch 1 oder 2, **dadurch gekennzeichnet, dass** die Peroxid - Verbindung Calciumperoxid ist.

4. Zahnpasta gemäss irgendeinem der vorhergehenden Ansprüche, **dadurch gekennzeichnet, dass** die Peroxid Konzentration, bezogen auf O₂²⁻, über 0.01% liegt.

5. Zahnpasta gemäss Anspruch 4, **dadurch gekennzeichnet, dass** die Konzentration der Peroxid-Verbindung mehr als 0.05% beträgt, vorzugsweise zwischen 0.1% und 10% liegt.

6. Zahnpasta gemäss Anspruch 4, **dadurch gekennzeichnet, dass** die Konzentration der Peroxid-Verbindung 0.1% beträgt.

7. Zahnpasta gemäss irgendeinem der vorhergehenden Ansprüche, **dadurch gekennzeichnet, dass** die Zahnpasta ein oder mehrere Enzyme aus der Klasse der Proteasen enthält.

8. Zahnpasta gemäss Anspruch 7, **dadurch gekennzeichnet, dass** die Proteasen aus der Klasse der Cystein Proteasen ausgewählt sind.

9. Zahnpasta gemäss Anspruch 8, **dadurch gekennzeichnet, dass** die Proteasen aus der Gruppe umfassend Papain und / oder Bromelain ausgewählt sind.

10. Zahnpasta gemäss irgendeinem der vorhergehenden Ansprüche, **dadurch gekennzeichnet, dass** sie Ubichinon und Vitamin E enthält.

11. Verwendung einer Zahnpasta gemäss irgendeinem der vorhergehenden Ansprüche zur Reinigung und optischen Aufhellung von natürlichen und/oder künstlichen Zähnen und Zahnfüllungen.

## Claims

1. Toothpaste comprising water; at least one abrasive and one or more micronized, non-encapsulated peroxide compounds, whereby 50% of the particles of said peroxide compound have a diameter of less than 3 microns and at least 90% of the particles of said peroxide compound have a diameter of less than 7 microns.

2. Toothpaste according to claim 1, **characterized in that** 50% of said particles have a diameter of less than 2 microns and at least 90% of said particles have a diameter of less than 5 microns.

3. Toothpaste according to claim 1 or 2, **characterized in that** said peroxide compound is calcium peroxide.

4. Toothpaste according to any of the preceding claims, **characterized in that** the concentration of peroxide is above 0.01%, based on O₂²⁻.

5. Toothpaste according to claim 4, **characterized in that** the concentration of peroxide is more than 0.05%, preferably is between 0.1% and 10%.

6. Toothpaste according to claim 4, **characterized in that** concentration of peroxide is 0.1%.

7. Toothpaste according to any of the preceding claims, **characterized in that**, said toothpaste comprises one or more enzymes from the class of proteases.

8. Toothpaste according to claim 7, **characterized in that** said proteases are selected from the group of cystein proteases.

9. Toothpaste according to claim 8, **characterized in that** said proteases are selected from the group comprising papain and / or bromelain.

10. Toothpaste according to any of the preceding claims, **characterized in that** it comprises ubichinone and vitamin E.

11. Use of a toothpaste according to any of the preceding claims for cleaning and optical brightening of natural and / or artificial teeth and dental fillings.

## Revendications

1. Pâte dentifrice contenant de l'eau ; au moins un agent abrasif et un ou plusieurs peroxydes micronisés et incapsulés, dans laquelle 50% des particules desdits peroxydes ont un diamètre de moins de 3 microns et au moins 90% des particules desdits peroxydes ont un diamètre de moins de 7 microns.

2. Pâte dentifrice suivant la revendication 1, **caractérisée en ce que** 50% desdites particules ont un diamètre de moins de 2 microns et au moins 90% desdites particules ont un diamètre de moins de 5 microns.

3. Pâte dentifrice suivant la revendication 1 ou 2, **caractérisée en ce que** le peroxyde est du peroxyde de calcium.

4. Pâte dentifrice suivant l'une quelconque des revendications précédentes, **caractérisée en ce que** le peroxyde a une concentration rapportée à O₂²⁻ supérieure à 0,01%.

5. Pâte dentifrice suivant la revendication 4, **caractérisée en ce que** la concentration du peroxyde est supérieure à 0,05%, en étant comprise, de préférence, entre 0,1% et 10%.

6. Pâte dentifrice suivant la revendication 4, **caractérisée en ce que** la concentration du peroxyde est de 0,1%.

7. Pâte dentifrice suivant l'une quelconque des revendications précédentes, **caractérisée en ce que** la pâte dentifrice contient un enzyme ou plusieurs enzymes de la classe des protéases.

8. Pâte dentifrice suivant la revendication 7, **caractérisée en ce que** les protéases sont choisies dans la classe des cystéine protéases.

9. Pâte dentifrice suivant la revendication 7, **caractérisée en ce que** les protéases sont choisies dans le groupe comprenant la papaïne et/ou la bromélaïne.

10. Pâte dentifrice suivant l'une quelconque des revendications précédentes, **caractérisée en ce qu'**elle contient de l'ubiquinone et de la vitamine E.

11. Utilisation d'une pâte dentifrice suivant l'une quelconque des revendications précédentes, pour le nettoyage et le blanchiment optique de dents naturelles et/ou artificielles et d'obturations dentaires.
